# EUROPÄISCHE PATENTSCHRIFT

(11) **EP 2 301 425 B1**
(45) Veröffentlichungstag und Bekanntmachung des Hinweises auf die Patenterteilung: **20.11.2019**
(21) Anmeldenummer: 09075450.8
(22) Anmeldetag: 29.09.2009
(51) Int. Cl.: A61B 3/12, A61F 9/008

(54) **Ophthalmoskop zum Beobachten eines Auges**
Ophthalmoscope for observing an eye
Ophtalmoscope pour l'observation d'un oeil

(43) Veröffentlichungstag der Anmeldung: 30.03.2011
(73) Patentinhaber: OD-OS GmbH, 14513 Teltow (DE)
(72) Erfinder: Amthor, Kay-Uwe, 14469 Potsdam (DE); Liesfeld, Ben, 14469 Potsdam (DE)
(74) Vertreter: Pfenning, Meinig & Partner mbB

(56) Entgegenhaltungen:
- WO-A1-91/01703
- DE-A1- 3 001 244
- DE-T2- 69 130 772
- US-A- 4 666 268
- J-M PAREL ET AL: "REVIEW ARTICLE; The optics of the ophthalmoscope and related instruments" JOURNAL OF PHYSICS E. SCIENTIFIC INSTRUMENTS, IOP PUBLISHING, BRISTOL, GB, Bd. 13, Nr. 12, 1. Dezember 1980 (1980-12-01), Seiten 1242-1253, XP020016042 ISSN: 0022-3735

## Beschreibung

Die Erfindung betrifft ein Ophthalmoskop zum Beobachten eines Auges nach Anspruch 1 und ein Verfahren zum Beobachten eines Auges nach Anspruch 13.

Die Beobachtung eines Auges, insbesondere eines Fundus oder Augenhintergrundes, dient in der Regel der Diagnose von Krankheiten des Auges sowie der Behandlung solcher Krankheiten. Zu diesen Krankheiten zählen insbesondere altersbedingte Makuladegenerationen oder diabetische Retinopathien. Eine frühzeitige Erkennung solcher Erkrankungen durch diagnostische Methoden, wie beispielsweise eine direkte Beobachtung der Retina (Netzhaut) durch einen Arzt, Fundusaufnahmen, Fluoreszenzaufnahmen oder optische Kohärenztomografien, bedürfen besonderer optischer Technologien, da der Augenhintergrund durch eine kleine Öffnung einer Augenpupille beobachtet bzw. abgebildet werden muss.

Zur Behandlung von Erkrankungen der Retina wird oftmals eine Laserbestrahlung an symptomatischen Bereichen der Retina durchgeführt. Für diese Behandlung muss eine Laserstrahlung durch die Augenpupille in das Auge eingekoppelt und auf der zu bestrahlenden Stelle fokussiert werden. Während einer solchen Behandlung ist ebenfalls die Beobachtung der Retina notwendig.

Zu diesem Zweck direkt am Auge eingesetzte optische Elemente spielen für eine Genauigkeit einer Abbildung des Augenhintergrundes eine entscheidende Rolle. Zur Beobachtung des Augenhintergrundes wird standardmäßig eine Spaltlampe verwendet. Mit einer Ophthalmoskopierlinse, wie sie beispielsweise in US 5,526,189 beschrieben wird, wird der Augenhintergrund in eine Zwischenbildebene abgebildet. DE 30 01 244 A1 und US 4 666 268 A beschrieben auch ähnliche Ophtalmoskope. Diese Zwischenbildebene wird mit einem Stereomikroskop betrachtet. Die Beleuchtung des Augenhintergrundes erfolgt durch eine Spaltbeleuchtung, die ebenfalls über die Ophthalmoskopierlinse in das Auge eingekoppelt wird. Entscheidende Nachteile einer solchen Fundusbeobachtung mit einer Spaltlampe ist zum einen eine Einschränkung auf ein spaltförmiges Sichtfeld auf der Retina, sowie Reflexionen der Beleuchtung an der Ophthalmoskopierlinse und einer Hornhaut (Kornea) des Patienten, die die Abbilddung des Fundus als störende Artefakte überlagern.

Des Weiteren ist es für eine Dokumentation einer Untersuchung bzw. Behandlung notwendig, Aufnahmen des Augenhintergrundes zu speichern. Zu diesem Zweck werden beispielsweise Funduskameras bzw. indirekte Ophthalmoskope verwendet. Ein sammelndes Linsensystem des Ophthalmoskops erzeugt dabei ein Zwischenbild des Augenhintergrunds, welches durch eine nachgelagerte Abbildungsoptik in einer Abbildungsebene abgebildet wird, wobei in dieser Abbildungsebene meistens ein elektronischer lichtempfindlicher Sensor angeordnet ist.

Folgende Parameter und Randbedingungen sind bei der Beobachtung des Augenhintergrundes mit einem indirekten Ophthalmoskop relevant. Ein Feldwinkel, welcher durch einen maximalen Winkel, den zwei vom Auge ausgehende und durch das Ophthalmoskop in der Abbildungsebene abbildbare Lichtstrahlen zwischen sich einschließen können, welcher möglichst groß sein sollte, um ein möglichst großes Sichtfeld des Augenhintergrundes zu ermöglichen, eine Größe des lichtempfindlichen Sensors und eine Baugröße der Abbildungsoptik des Ophthalmoskops, welches möglichst kompakt sein sollte, um den Abstand zwischen Arzt und dem zu untersuchenden Patienten möglichst gering zu halten (kleiner als eine Armlänge). Relevante Parameter sind außerdem eine möglichst hohe Abbildungsqualität, ein Durchmesser der Pupille des untersuchten Auges, welcher bei einer vorzugsweise nichtmydriatitische Untersuchung in der Regel kleiner als 4 mm ist, ein Arbeitsabstand zwischen dem Ophthalmoskop und dem Auge, definiert durch einen Abstand zwischen der Kornea des Auges und einer dem Auge zugewandten Oberfläche einer Eingangslinse des Ophthalmoskops, welcher möglichst groß sein sollte, damit weitere optische Elemente zwischen Ophthalmoskop und Auge möglichst problemlos platziert werden können und ferner ein größerer Abstand zwischen dem Ophthalmoskop und einer Nase und einer Stirn des Patient erzielt wird, um eine größere Bewegungsfreiheit für das Ophthalmoskop zu erzielen. Die genannten Anforderungen sind eng miteinander verknüpft, so dass Änderungen eines dieser Parameter Auswirkungen auf die anderen Parameter hat.

Wählt man beispielsweise einen kostengünstigen Sensor mit einer Diagonale von etwa 0,5 Zoll oder kleiner, so muss ein Zwischenbild des Augenhintergrunds in der Regel stark verkleinert auf diesen Sensor abgebildet werden. Dies erfordert entweder eine große Baulänge der Abbildungsoptik, wodurch sich der Abstand zwischen Arzt und Patienten vergrößert, oder den Einsatz optischer Elemente mit kurzen Brennweiten, welche allerdings dem Erreichen einer hohen Abbildungsqualität entgegensteht. Um einen möglichst großen Arbeitsabstand zu erzielen, kann man ein Objektiv des Ophthalmoskops mit einer großen Brennweite wählen. Da aber die Größe des Zwischenbildes ebenfalls mit der Brennweite des Objektivs skaliert, lässt sich das größere Zwischenbild bei unveränderter nachgelagerter Optik nicht mehr vollständig vom Sensor erfassen. Daher muss entweder ein größerer Sensor gewählt werden, was die Kosten des Ophthalmoskops stark erhöht oder die nachgelagerte Optik muss wiederum wie oben beschrieben eine starke Verkleinerung des Zwischenbildes erzielen können, was entweder eine längere Bauform oder den Einsatz stark brechender optischer Elemente erfordert mit oben genannten Nachteilen.

Neben der Erzeugung eines Zwischenbildes hat das Objektiv eines Ophthalmoskops außerdem die Aufgabe, eine Maxwellsche Beleuchtung zu ermöglichen, bei der eine Gerätepupille, welche in der Regel durch eine Apertur der Abbildungsoptik gegeben ist, in die Pupille des zu untersuchenden Auges abgebildet wird. Die Maxwellsche Beleuchtung, welche auch als Pupillenabbildung bezeichnet wird, ermöglicht eine räumliche Trennung eines Beobachtungsstrahlenganges und eines Beleuchtungsstrahlenganges an der Hornhaut des Auges in der Nähe der Augenpupille. Durch diese räumliche Trennung der genannten Strahlengänge wird gewährleistet, dass Reflexionen an der Hornhaut des Auges und an Oberflächen des Objektivs in der Fundusaufnahme nicht die Reflexion vom Augenhintergrund, also den Beobachtungsstrahl, der die entscheidenden Bildinformation vom Auge trägt, überlagert. Außerdem ermöglicht die Pupillenabbildung einen besonders großen Feldwinkel, da ein besonders großer Bereich des Auges auf dem Sensor des Ophthalmoskops abgebildet werden kann. Die Pupillenabbildung setzt allerdings einen korrekt eingestellten Arbeitsabstand voraus. Wird das Ophthalmoskop etwa zu weit von dem Auge entfernt positioniert, wird die Apertur vor dem Auge außerhalb der Pupille abgebildet. Dadurch verringert sich der Feldwinkel und werden außerdem Streulichtanteile nicht so wie oben beschrieben aus dem Beobachtungsstrahlengang entfernt.

Der vorliegenden Erfindung liegt also die Aufgabe zugrunde, ein Ophthalmoskop zur Beobachtung eines Auges, insbesondere eines Augenhintergrundes, vorzuschlagen, mit dem sich ein großer Arbeitsabstand und großer Feldwinkel erzielen lässt, wobei aber die Baulänge des Ophthalmoskops möglichst kompakt sein soll und wobei gleichzeitig eine möglichst gute Pupillenabbildung erzielt werden soll. Ferner soll eine möglichst hohe Bildqualität mit einem möglichst kleinen Sensor erzielt werden. Außerdem soll ein entsprechend vorteilhaftes Verfahren vorgeschlagen werden zur Beobachtung eines Auges.

Diese Aufgabe wird erfindungsgemäß gelöst durch ein Ophthalmoskop und ein Verfahren mit den Merkmalen der unabhängigen Ansprüche. Vorteilhafte Ausführungsformen der Erfindung sind Gegenstand der Unteransprüche.

Ein erfindungsgemäßes Ophthalmoskop zum Beobachten eines Auges umfasst ein sammelndes (positiv brechendes) erstes Linsensystem zur Erzeugung eines reellen Zwischenbildes eines Bereichs im Auge in einer Zwischenbildebene sowie eine Beobachtungsvorrichtung mit einer Abbildungsoptik zum Abbilden des reellen Zwischenbildes in einer Abbildungsebene in der Beobachtungsvorrichtung und zum Abbilden einer Apertur der Beobachtungsvorrichtung in einer Pupille des Auges, wobei zwischen dem ersten Linsensystem und der Abbildungsoptik der Beobachtungsvorrichtung ein streuendes (negativ brechendes) zweites Linsensystem vorgesehen ist zur Vergrößerung eines Arbeitsabstandes zwischen dem ersten Linsensystem und der Pupille und zur Vergrößerung eines Feldwinkels.

Dabei umfasst das erste Linsensystem mindestens eine sammelnde (positiv brechende) Linse und umfasst ferner das zweite Linsensystem mindestens eine streuende (negativ brechende) Linse. Beide Linsensystem können auch weitere Linsen umfassen.

Unter einem sammelnden (positiv brechenden) Linsensystem wird ein System von mindestens einer Linse verstanden, welches einen Strahlenbündel parallel zueinander verlaufender Lichtstrahlen, der durch das positiv brechende Linsensystem hindurch läuft, bündelt. Unter einem negativ brechenden Linsensystem ist ein System von mindestens einer Linse zu verstehen, welches einen solchen Strahlenbündel, der dieses Linsensystem durchläuft, auseinander bricht und zerstreut.

Durch die erfindungsgemäße Anordnung des streuenden zweiten Linsensystems wird erreicht, dass der zur Erzielung der Pupillenabbildung notwendige Arbeitsabstand, wie oben definiert durch einen Abstand zwischen der Kornea des Auges und einer dem Auge zugewandten Oberfläche einer dem Auge zugewandten Eingangslinse des ersten Linsensystems, vergrößert wird. Insbesondere ist der durch die Erfindung erzielbare Arbeitsabstand größer als der Abstand zwischen dieser Oberfläche und einem dem Auge zugewandten Brennpunkt des ersten Linsensystems, welcher dem Arbeitsabstand eines gewöhnlichen Ophthalmoskops ohne das zweite Linsensystem entspricht. Ein vergrößerter Arbeitsabstand hat, wie bereits erwähnt, den Vorteil, dass zwischen dem Ophthalmoskop und dem Auge leicht weitere Geräte, wie Beispielsweite eine Kontaktlinse, platziert werden können. Ferner ist ein größerer Feldwinkel erzielbar und entsteht ein größerer Bewegungsspielraum für das Ophthalmoskop, da insbesondere auch der Abstand zu einer Nase und einer Stirn des Patienten vergrößert wird.

In einer Weiterentwicklung, welche sich durch besonders gute Abbildungseigenschaften auszeichnet, ist vorgesehen, dass die Zwischenbildebene innerhalb des zweiten Linsensystems, in einem ersten Zwischenraum zwischen dem ersten Linsensystem und dem zweiten Linsensystem oder in einem zweiten Zwischenraum zwischen dem zweiten Linsensystem und der Abbildungsoptik angeordnet ist.

Falls das zweite Linsensystem genau eine Linse beinhaltet, so befindet sich die Zwischenbildebene innerhalb des zweiten Linsensystems, wenn die Zwischenbildebene die betreffende Linse durchläuft. Falls das zweite Linsensystem mehrere Linsen umfasst, befindet sich die Zwischenbildebene innerhalb des zweiten Linsensystems, falls sich die Zwischenbildebene zwischen diesen beiden Linsen befindet oder mindestens eine dieser beiden Linsen durchläuft. Analoges gilt für das erste Linsensystem und die Abbildungsoptik. Die Zwischenbildebene liegt in dem ersten Zwischenraum zwischen dem ersten und dem zweiten Linsensystem, falls sie sich zwischen einer dem ersten Linsensystem zugewandten Oberfläche des zweiten Linsensystems und einer dem zweiten Linsensystem zugewandten Oberfläche des ersten Linsensystems befindet, aber sich weder innerhalb des ersten noch des zweiten Linsensystems befindet. Die Zwischenbildebene liegt in dem zweiten Zwischenraum zwischen dem zweiten Linsensystem und der Abbildungsoptik, falls sie sich zwischen einer der Abbildungsoptik zugewandten Oberfläche des zweiten Linsensystems und einer dem zweiten Linsensystem zugewandten Oberfläche der Abbildungsoptik befindet, aber sich weder innerhalb des zweiten Linsensystems noch innerhalb der Abbildungsoptik befindet.

In einer Ausführungsform ist vorgesehen, dass das zweite Linsensystem genau eine streuende Linse umfasst und die Zwischenbildebene innerhalb dieser Linse angeordnet ist. Durch den Einsatz einer einzelnen streuenden Linse in der Zwischenbildebene wird ein besonders großer Arbeitsabstand erzielen. Andererseits bleibt die Brennweite des ersten Linsensystems für die Abbildung des Zwischenbildes in der Zwischenbildebene unverändert, so dass sich erstens die Zwischenbildebene durch das zweite Linsensystem nicht verlagert und zweitens das Zwischenbild nicht vergrößert wird, so dass die nachgelagerte Abbildungsoptik nicht an das zweite Linsensystem angepasst werden muss.

Eine alternative Ausführungsform des erfindungsgemäßen Ophthalmoskops sieht vor, dass die Zwischenbildebene in dem ersten Zwischenraum in unmittelbarer Nähe zu einer dem ersten Linsensystem zugewandten Oberfläche des zweiten Linsensystems angeordnet ist oder dass die Zwischenbildebene in dem zweiten Zwischenraum in unmittelbarer Nähe zu einer der Abbildungsoptik zugewandten Oberfläche des zweiten Linsensystems angeordnet ist. Auf diese Weise lässt sich eine Strahlungsleistung innerhalb des zweiten Linsensystems reduzieren. Dies hat insbesondere bei einer Laserbehandlung des Auges den Vorteil, dass das zweite Linsensystem vor einer zu hohen Strahlungsintensität des Lasers, welche innerhalb der Zwischenbildebene besonders groß ist, geschützt wird. Insbesondere zur Behandlung des Augenhintergrundes wird ein Therapielaserstrahl nämlich auf den Augenhintergrund fokussiert zur Erzielung einer möglichst hohen Strahlungsleistung im Augenhintergrund. Eine Fokussierung des Laserstrahls auf den Augenhintergrund bedeutet gleichzeitig eine Fokussierung des Lasers auf die Zwischenbildebene.

Um einen gleichzeitig möglichst großen Arbeitsabstand zwischen dem Auge und dem ersten Linsensystem zu erzielen, die Lage der Zwischenbildebene nicht zu verändern und das Zwischenbildes möglichst nicht zu vergrößern, ist in einem Ausführungsbeispiel vorgesehen, dass dass die Zwischenbildebene in dem ersten Zwischenraum in einem Abstand von 1 mm bis 20 mm zu einer dem ersten Linsensystems zugewandten Oberfläche des zweiten Linsensystems angeordnet ist oder dass die Zwischenbildebene in dem zweiten Zwischenraum in einem Abstand von 1 mm bis 20 mm zu einer der Abbildungsoptik zugewandten Oberfläche des zweiten Linsensystems angeordnet ist. In einer weiteren Ausführungsbeispiel liegt der genannte Abstand zwischen 5 Millimetern und 15 Millimetern.

Eine Ausführungsform des Ophthalmoskops hier vorgestellter Art sieht vor, dass das zweite Linsensystem mindestens zwei Linsen beinhaltet, wobei die Zwischenbildebene in einem Zwischenraum zwischen den mindestens zwei Linsen angeordnet ist. Auf diese Weise lässt sich wiederum eine Reduzierung einer Strahlungsleistung eines Therapielaserstrahls innerhalb eines Linsenmaterials des zweiten Linsensystems reduzieren und gleichzeitig ein großer Arbeitsabstand erzielen.

In einer weiteren Ausgestaltung der Erfindung ist mindestens eine in dem zweiten Linsensystem enthaltene Linse relativ zur Zwischenbildebene gekippt. Auf diese Weise lässt sich ein Streulichtanteil aus dem Beobachtungsstrahl entfernen und eine Abbildungsqualität des Ophthalmoskops verbessern. Vorzugsweise liegt ein entsprechender Drehwinkel zwischen der gekippten Linse und der Zwischenbildebene in einem Bereich zwischen 0° und 45 °, in einer Weiterentwicklung liegt der Drehwinkel innerhalb eines Bereiches zwischen 5° und 15°. Alternativ oder zusätzlich können auch in dem ersten Linsensystem enthaltene Linsen entsprechend verkippt sein.

In einer Ausführungsform der Erfindung ist vorgesehen, dass das erste Linsensystem eine Linse mit einer gekrümmten Oberfläche aufweist, wobei die Oberfläche asphärisch ausgebildet ist. Dies dient dem Zweck, eine Optimierung der Pupillenabbildung und der Fundusabbildung mittels einer möglichst geringen Anzahl optischer Flächen. Eine solche Optimierung ist mittels asphärischer Oberflächen besonders vorteilhaft, da eine asphärische Ausformung der Linsenoberfläche eine lokale Optimierung für einen jeden Betrachtungswinkel ermöglicht. Dabei können lokale Krümmungsradien der aspährischen Oberfläche entsprechend einer Pfeilhöhe eines Strahlenbündels angepasst werden. Dabei ist die Pfeilhöhe des Strahlenbündels definiert als der Abstand zwischen einer Symmetrieachse (bezüglich einer Rotationssymmetrie) der Linse und einem Eintrittspunkt des Strahlenbündels in die Linse.

Die Optimierung der Abbildungseigenschaften Ophthalmoskops mittels asphärischer Linsenoberflächen hat gegenüber einer Optimierung mittels zusätzlicher sphärischer Linsen den Vorteil einer Einsparung zusätzlicher Linsenoberflächen (optischer Flächen) und damit einer Vermeidung zusätzlicher Lichtreflexe an diesen Oberflächen.

In einer alternativen Ausführungsform der Erfindung ist vorgesehen, dass das zweite Linsensystem eine Linse mit einer gekrümmten Oberfläche aufweist, wobei diese Oberfläche asphärisch ausgeformt ist. Die asphärische Ausformung dieser Linse dient wiederum dem oben beschriebenen Zweck. In einer bevorzugten Ausführungsform weisen sowohl das erste wie auch das zweite Linsensystem jeweils eine Linse mit einer derartig asphärisch ausgeformten Oberfläche auf.

In einer Weiterentwicklung der Erfindung ist vorgesehen, dass die Beobachtungsvorrichtung einen lichtempfindlichen Sensor aufweist, der in der Abbildungsebene angeordnet ist, zum Erzeugen elektrischer Bildsignale. Ein solcher Sensor hat die Eignung, eine digitale Bildverarbeitung zu ermöglichen. Ein solches mit einer digitalen Bildverarbeitung ausgestattetes Ophthalmoskop bietet eine Vielzahl diagnostischer Zusatzfunktionen, welche hier im Einzelnen nicht weiter erläutert werden sollen.

Eine vorteilhafte Ausführungsform des Ophthalmoskops sieht vor, dass das Ophthalmoskop im Zwischenbereich zwischen dem ersten und dem zweiten Linsensystem keinen Spiegel, keinen Strahlteiler und/oder keine weitere Linse beinhaltet. Auf diese Weise ist ein möglichst einfacher und kostengünstiger Aufbau realisierbar, welcher zudem sich durch eine besonders hohe Bildqualität auszeichnet, da durch eine Einsparung optischer Flächen eine Reduzierung von Streulicht und unerwünschter Lichtreflexe erzielt wird. Aus demselben Grund sieht eine weitere Ausführungsform der Erfindung vor, dass das Ophthalmoskop in einem Zwischenraum zwischen dem Auge und dem ersten Linsensystem keinen Spiegel, keinen Strahlteil und/oder keine weitere Linse umfasst.

Eine besonders einfache Ausführungsform der Erfindung, welche sich durch eine hohe Abbildungsqualität und einen geringen Streulichtanteil auszeichnet, sieht vor, dass neben einer Abbildung des Bereichs im Auge in der Zwischenbildebene und in der Abbildungsebene keine weiteren Abbildungen des Auges vorgesehen sind.

Das erfindungsgemäße Verfahren zum Beobachten eines Auges mit einem Ophthalmoskops hier vorgestellter Art sieht vor, dass ein im Auge reflektierter Beobachtungsstrahl durch ein erstes Linsensystem des Ophthalmoskops als Zwischenbild in einer Zwischenbildebene abgebildet wird und das Zwischenbild durch eine Abbildungsoptik einer Beobachtungsvorrichtung des Ophthalmoskops in einer Abbildungsebene abgebildet wird, wobei der Beobachtungsstrahl in einem Strahlenverlauf zwischen dem ersten Linsensystem und der Abbildungsoptik durch ein zweites Linsensystem gestreut wird und wobei ferner eine Apertur der Beobachtungsvorrichtung in einer Pupille des Auges abgebildet wird. In einer Ausführungsform wird dieses Verfahren mit einem Ophthalmoskop hier vorgeschlagener Art durchgeführt.

Im Folgenden wird die Erfindung anhand spezieller Ausführungsbeispiele näher beschrieben. Es zeigt:
- Figur 1: eine Pupillenabbildung mittels eines Ophthalmoskops hier vorgeschlagener Art,
- Figur 2: eine Abbildung eines Augenhintergrundes mittels eines Ophthalmoskops hier vorgeschlagener Art,
- Figur 3: Ophthalmoskop hier vorgeschlagener Art mit einer Zwischenbildebene innerhalb einer Linse des zweiten Linsensystems,
- Figur 4: ein Ophthalmoskop hier vorgeschlagener Art mit einer relativ zur Zwischenbildebene verkippten Linse,
- Figur 5: Ophthalmoskop hier vorgeschlagener Art mit einer Zwischenbildebene in einem ersten Zwischenraum zwischen dem ersten und dem zweiten Linsensystem,
- Figur 6: Ophthalmoskop hier vorgeschlagener Art mit einer Zwischenbildebene innerhalb des zweiten Linsensystems.

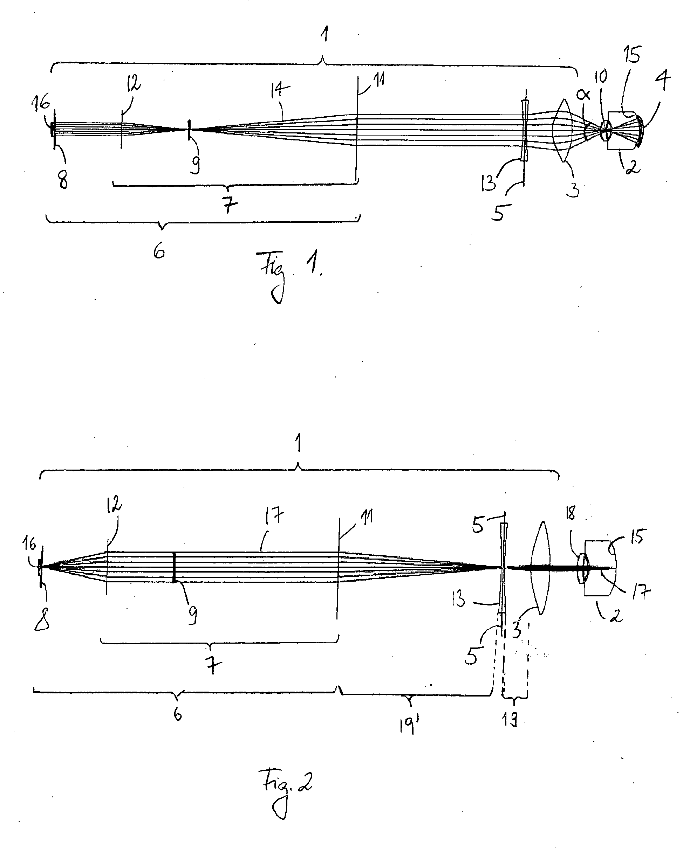

Figur 1 zeigt eine schematische Darstellung eines speziellen Ausführungsbeispiels eines Ophthalmoskops 1 hier vorgeschlagener Art zum Beobachten eines Auges 2. Das Ophthalmoskop 1 umfasst ein sammelndes erstes Linsensystem 3 zur Erzeugung eines reellen Zwischenbildes 5 eines Bereichs 4 im Auge in einer Zwischenbildebene 5 sowie eine Beobachtungsvorrichtung 6 mit einer Abbildungsoptik 7 zum Abbilden des reellen Zwischenbildes 5 in einer Abbildungsebene 8 innerhalb der Beobachtungsvorrichtung 6 und zum Abbilden einer Appertur 9 der Beobachtungsvorrichtung 6 in einer Pupille des Auges 4. Die Abbildungsoptik 7 beinhaltet in diesem speziellen Beispiel zwei sammelnden Linsen 11, 12. In komplexeren Ausführungsformen sind zur Erzielung einer höheren Abbildungsqualität anstelle der einzelnen Linsen 11 und 12 jeweils Linsensysteme vorgesehen, welche mehrere Linsen umfassen können.

Das Ophthalmoskop weist ferner ein streuendes zweites Linsensystem 13 auf, wobei die Zwischenbildebene 5 innerhalb dieses zweiten Linsensystems 13 verläuft. In dem hier dargestellten Ausführungsbeispiel beinhaltet das zweite Linsensystem 13 eine einzige streuende Linse 13.

Der in Figur 1 schematisch dargestellte Strahlenverlauf dient der Illustration einer Pupillenabbildung, also der Abbildung der Apertur 9 auf die Augenpupille 10, wobei ein von der Apertur 9 ausgehendes divergentes Strahlenbündel 14 auf die Pupille 10 fokussiert wird. Durch die Pupillenabbildung wird ein besonders großer Bereich 4 im Auge ausgeleuchtet bzw. beobachtet. Einer Größe des Bereichs 4 entspricht unmittelbar einem großen Feldwinkel a. In dem hier dargestellten Ausführungsbeispiel beträgt der Feldwinkel 50°, was einem Bereich 4 mit einem Durchmesser von ca. 16 Millimetern auf einem Hintergrund des Auges 15 entspricht.

In diesem Ausführungsbeispiel beinhaltet das erste sammelnde Linsensystem 3 eine einzige sammelnde Linse 3, welche also die Eingangslinse des Ophthalmoskops 1 darstellt, welche asphärisch ausgeformt ist zum Verbessern von Abbildungseigenschaften des Ophthalmoskops unter großen Beobachtungswinkeln (Feldwinkeln) α. Das Ophthalmoskop 1 beinhaltet ferner einen lichtempfindlichen Sensor 16, welcher in der Abbildungsebene 8 angeordnet ist, zum Erzeugen elektrischer Bildsignale. Ferner beinhaltet das Ophthalmoskop eine digitale Bildverarbeitungseinheit (hier nicht dargestellt) zur Durchführung von Bildverarbeitungsfunktionen.

In Figur 2 ist das bereits oben anhand von Figur 1 beschriebene beispielhafte Ophthalmoskop 1 erneut dargestellt. In Figur 2 ist die Situation einer Fundusabbildung illustriert, die sich dadurch auszeichnet, dass ein von dem Augenhintergrund 15 ausgehendes Strahlenbündel durch das erste sammelnde Linsensystem 3 in der Zwischenbildebene 5 abgebildet wird. Im weiteren Strahlenverlauf wird dieses Strahlenbündel durch die erste sammelnde Linse 11 der Abbildungsoptik 7 parallelisiert, durch die Apertur 9 (Aperturblende) räumlich begrenzt, und anschließend durch die zweite Sammellinse 12 der Abbildungsoptik 7 in der Abbildungsebene 8 auf den Sensor 16 abgebildet.

Das erste Linsensystem 2 und das zweite Linsensystem 13 des hier beschriebenen Ophthalmoskops 1 haben somit die Eignung gleichzeitig den Augenhintergrund 15 des Auges 2 auf den Sensor 16 der Beobachtungsvorrichtung 6 abzubilden und die Apertur 9 in der Pupille 10 abzubilden, welche sich hinter der Hornhaut 18 des Auge 2 innerhalb des Auges 2 befindet.

Durch die im zweiten Linsensystem enthaltene Streulinse 13 wird zudem ein Arbeitsabstand zwischen dem ersten Linsensystem und dem Auge 2 vergrößert, wie anhand der folgenden Figuren näher erläutert wird.

In diesem Ausführungsbeispiel ist die Zwischenbildeben 5 innerhalb des Linsensystem 13 angeordnet. In alternativen Ausführungsformen befindet sich die Zwischenbildebene (5) in einem ersten Zwischenraum (19) zwischen dem ersten Linsensystem (3) und dem zweiten Linsensystem (13) oder in einem zweiten Zwischenraum (19') zwischen dem zweiten Linsensystem (13) und der Abbildungsoptik (7)

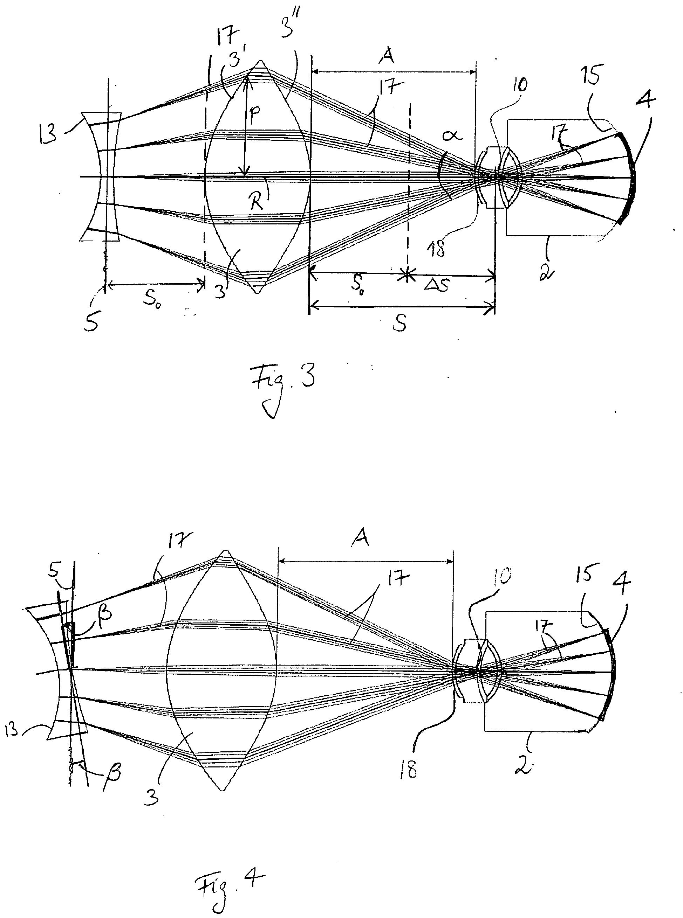

In Figur 3 ist ein Ausschnitt eines anhand von Figuren 1 und 2 beschriebenen Ophthalmoskops dargestellt. In Figur 3 sind die Strahlenverläufe mehrerer von dem beobachteten Bereich 4 auf dem Hintergrund 15 des Auges 1 ausgehende Strahlenbündel 17 eingezeichnet. Ausgehend von dem Augenhintergrund 15 durchlaufen sie zunächst die Pupille 10 und treten aus dem Auge 1 aus. Anschließend treffen sie zunächst auf das sammelnde Linsensystem 3 (der Eingangslinse), welches sie in der Zwischenbildebene 5 fokussiert.

Die aus dem Auge 4 austretenden Strahlenbündel 17 bestehen aus kollimierten Lichtstrahlen, so dass die einzelnen Strahlenbündel durch das sammelnde Linsensystem 3 in einer mit der Zwischenbildebene 5 identischen Brennebene 5 des ersten Linsensystems 3 fokussiert werden. Somit entspricht ein als S₀ beschrifteter Abstand zwischen der Zwischenbildebene 5 und einer der Zwischenbildebene 5 zugewandten Oberfläche 3' des sammelnden Linsensystems 3 etwa einer Brennweite des Linsensystems 3.

Die Sammellinse 3 ist ferner, wie bereits oben beschrieben, asphärisch ausgeformt. Auf diese Weise werden auch solche Strahlenbündel 17, welche mit einer hohen Pfeilhöhe P, auf die Sammellinse 3 auftreffen, auf die Zwischenbildebene 5 abgebildet, so dass möglichst der gesamte Bereich 4 mit der Zwischenbildebene 5 optisch konjugiert ist. Dabei ist die Pfeilhöhe als der Abstand zwischen dem Eintrittspunkt des betreffenden Lichtbündels und einer Symmetrieachse R der Linse definiert.

Durch die Anordnung der Streulinse 13 in der Zwischenbildebene 5 wird Arbeitsabstand A zwischen einer dem Augen zugewandten Oberfläche 3'' des ersten Linsensystems 3 und einer Hornhaut 18 des Auges vergrößert. Ohne diese Streulinse 13 entspräche der Arbeitsabstand, welcher für eine Pupillenabbildung notwendig ist, etwa dem Abstand S₀ zwischen der der Zwischenbildebene 5 zugewandten Oberfläche 3' des Linsensystems 3. Durch die Hinzunahme des streuenden Linsensystems 13 werden die Strahlenbündel 17 in einem Abstand S, einer Schnittweite des Ophthalmoskops, von dem ersten Linsensystem gebündelt, welcher um einen Wert ΔS größer ist als S₀. Damit ergibt sich ein größerer Arbeitsabstand A zwischen der dem Auge 1 zugewandten Oberfläche 3'' der Sammellinse 3 und der Hornhaut 18 des Auges. Ein solcher vergrößerter Arbeitsabstand A ermöglicht es einem Beobachter des Auges 2 das Ophthalmoskop ohne Einschränkung einer Nase oder eines Stirnbeins vor dem Auge 1 zu bewegen.

In Figur 4 ist wiederum ein Ausschnitt eines speziellen Ausführungsbeispiels eines Ophthalmoskops hier vorgestellter Art ausschnittsweise und schematisch dargestellt. Der Unterschied zu dem anhand von Figur 3 beschriebenen Ophthalmoskops besteht in einer Verkippung des streuenden zweiten Linsensystems 13 relativ zur Zwischenbildebene 5 um einen Winkel β. Im vorliegenden Fall beträgt der Winkel β der Verkippung 9,5°. Die Verkippung hat die positive Wirkung einer Entfernung von Lichtreflexen aus dem Strahlengang des Ophthalmoskops, so dass diese Lichtreflexe nicht zu einer Verminderung einer Bildqualität einer Abbildung des Bereichs 4 führen können.

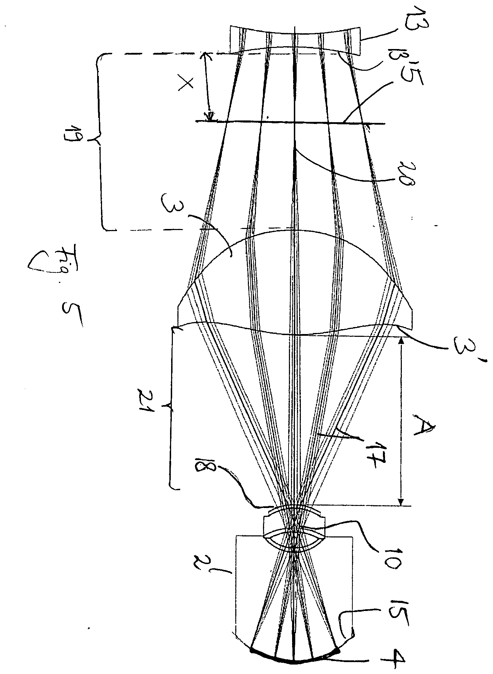

In Figur 5 ist wiederum eine spezielle Ausführungsform eines Ophthalmoskops hier vorgeschlagener Art ausschnittsweise und schematisch dargestellt. Im Unterschied zu den anhand von Figuren 1 bis 4 beschriebenen Ausführungsformen ist in diesem Fall die Zwischenbildebene 5 in einem ersten Zwischenraum 19 zwischen dem ersten Linsensystem 3 und dem zweiten Linsensystem 13 angeordnet. Ein Abstand X zwischen der Zwischenbildebene 5 und einer der Zwischenbildebene zugewandten Oberfläche 13' der zweiten Linsensystems 13 beträgt im vorliegenden Beispiel etwa 2 Millimeter. Dies hat den Vorteil, dass im Fall einer Laserbehandlung des Augenhintergrundes 15 des Auges 2 eine Strahlungsleistung innerhalb des zweiten Linsensystems 13 im Vergleich zu einer Strahlungsintensität innerhalb der Zwischenbildebene 5 verringert wird. Ferner ist die Oberfläche aspährisch ausgeformt. Ferner sind in einem Zwischenbereich 21 zwischen dem ersten Linsensystem 3 und dem Auge 2 keine weiteren Spiegel, Strahlteiler oder Linsen vorgesehen.

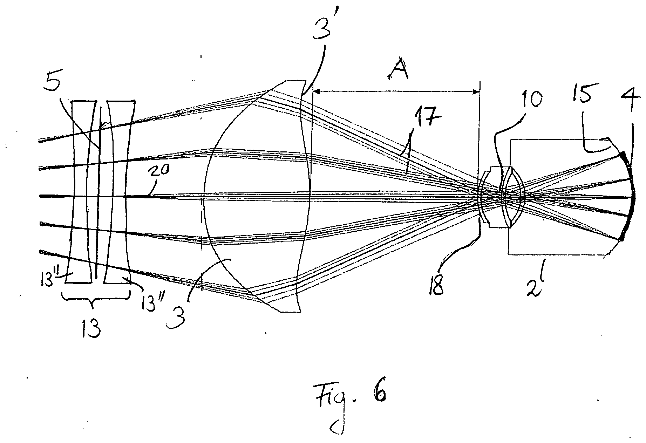

In Figur 6 ist ein weiteres Ausführungsbeispiel eines Ophthalmoskops hier vorgeschlagener Art ausschnittsweise und schematisch dargestellt. Im Unterschied zum anhand von Figur 5 beschriebenen Ophthalmoskops, beinhaltet das zweite Linsensystem 13 dieses Ausführungsbeispiels zwei Streulinsen 13''. Die Zwischenbildebene 5 ist in einem Zwischenbereich zwischen den beiden Streulinsen 13'' innerhalb des zweiten Linsensystems 13 angeordnet. Dies hat wiederum den Vorteil einer Reduzierung einer Strahlungsleistung innerhalb der Streulinsen 13'' des zweiten Linsensystems 13 zum Schutz dieser Linsen 13'' vor einer zu hohen Strahlungsintensität etwa im Fall einer Laserbehandlung des Augenhintergrunds 15.

Die hier genannten Abstände S₀, ΔS,S und X sind jeweils entlang einer optischen Achse 20 des Ophthalmoskops definiert.

### Bezugszeichenliste:

- 1: Ophthalmoskop
- 2: Auge
- 3: erstes Linsensystem
- 3': der Zwischenbildebene zugewandte Oberfläche des ersten Linsensystems
- 3": dem Auge zugewandte Oberfläche des ersten Linsensystems
- 4: Bereich des Auges
- 5: Zwischenbildebene
- 6: Beobachtungsvorrichtung
- 7: Abbildungsoptik
- 8: Abbildungsebene
- 9: Apertur
- 10: Pupille
- 11: erste Linse der Abbildungsoptik
- 12: zweite Linse der Abbildungsoptik
- 13: zweites Linsensystem
- 14: Beobachtungsstrahl
- 15: Augenhintergrund
- 16: Sensor
- 17: Beobachtungsstrahl
- 18: Hornhaut
- 19: Zwischenraum
- 20: Optische Achse
- 21: Zwischenraum

- α: Feldwinkel
- β: Verkippungswinkel
- A: Arbeitsabstand
- S: Schnittweite
- S₀: Abstand zwischen Zwischenbildebene und erstem Linsensystem
- ΔS: Differenz zwischen S und s0
- R: Symmetrieachse des ersten Linsensystems,
- p: Pfeilhöhe

## Patentansprüche

1. Ophthalmoskop (1) zum Beobachten eines Auges (2), insbesondere eines Hintergrundes (15) des Auges (2), umfassend ein sammelndes erstes Linsensystem (3) zur Erzeugung eines reellen Zwischenbildes eines Bereichs (4) im Auge (2) in einer Zwischenbildebene (5) sowie eine Beobachtungsvorrichtung (6) mit einer Abbildungsoptik (7) zum Abbilden des reellen Zwischenbildes in einer Abbildungsebene (8) in der Beobachtungsvorrichtung (6) und zum Abbilden einer Apertur (9) der Abbildungsoptik (7) in einer Pupille (10) des Auges (2),
**dadurch gekennzeichnet, dass**
zwischen dem ersten Linsensystem (3) und der Abbildungsoptik (7) der Beobachtungsvorrichtung (6) ein streuendes zweites Linsensystem (13) so angeordnet ist, dass sich die Zwischenbildebene (5) innerhalb des zweiten Linsensystems (13) in einem ersten Zwischenraum (19) zwischen dem ersten Linsensystem (3) und dem zweiten Linsensystem (13) in unmittelbarer Nähe zu einer dem ersten Linsensystem (3) zugewandten Oberfläche (13') des zweiten Linsensystems (13) oder in einem zweiten Zwischenraum (19') zwischen dem zweiten Linsensystem (13) und der Abbildungsoptik (7) in unmittelbarer Nähe zu einer der Abbildungsoptik (7) zugewandten Oberfläche (13'') des zweiten Linsensystems (13) befindet.

2. Ophthalmoskop (1) aus Anspruch 1, **dadurch gekennzeichnet, dass** das zweite Linsensystem mindestens zwei Linsen beinhaltet, wobei die Zwischenbildebene in einem Zwischenraum zwischen den mindestens zwei Linsen angeordnet ist.

3. Ophthalmoskop (1) nach Anspruch 1, **dadurch gekennzeichnet, dass** das zweite Linsensystem (13) genau eine streuende Linse (13) umfasst und die Zwischenbildebene (5) innerhalb dieser Linse (13) angeordnet ist.

4. Ophthalmoskop (1) nach Anspruch 1, **dadurch gekennzeichnet, dass** die Zwischenbildebene (5) in dem ersten Zwischenraum (19) in einem Abstand von 1 mm bis 20 mm zu einer dem ersten Linsensystems zugewandten Oberfläche (13') des zweiten Linsensystems (13) angeordnet ist oder dass die Zwischenbildebene (5) in dem zweiten Zwischenraum (19') in einem Abstand von 1 mm bis 20 mm zu einer der Abbildungsoptik (7) zugewandten Oberfläche (13') des zweiten Linsensystems angeordnet ist zum Reduzieren einer Strahlungsleistung innerhalb des zweiten Linsensystems (13) bei einem gleichzeitig großen Arbeitsabstand (A).

5. Ophthalmoskop (1) nach einem der vorangehenden Ansprüche, **dadurch gekennzeichnet, dass** mindestens eine in dem zweite Linsensystem (13) enthaltene Linse (13) relativ zur Zwischenbildebene (5) gekippt ist zum Unterdrücken von Streulicht.

6. Ophthalmoskop (1) nach einem der vorangehenden Ansprüche, **dadurch gekennzeichnet, dass** mindestens eine in dem zweiten Linsensystem enthaltene Linse relativ zur Zwischenbildebene (5) um einen Winkel (β) in einem Bereich zwischen 0° und 45° gekippt ist.

7. Ophthalmoskop (1) nach einem der vorangehenden Ansprüche, **dadurch gekennzeichnet, dass** das erste Linsensystem (3) eine Linse (3) mit einer gekrümmten Oberfläche (3') aufweist, die asphärisch ausgeformt ist zum Verbessern von Abbildungseigenschaften des ersten Linsensystems.

8. Ophthalmoskop (1) nach einem der vorangehenden Ansprüche, **dadurch gekennzeichnet, dass** das zweite Linsensystem (13) eine Linse (13) mit einer gekrümmten Oberfläche (13') aufweist, die asphärisch ausgeformt ist zum Verbessern von Abbildungseigenschaften des zweiten Linsensystems.

9. Ophthalmoskop (1) nach einem der vorangehenden Ansprüche, **dadurch gekennzeichnet, dass** die Beobachtungsvorrichtung (6) einen lichtempfindlichen Sensor (16) aufweist, der in der Abbildungsebene (8) angeordnet ist, zum Erzeugen elektrischer Bildsignale.

10. Ophthalmoskop (1) nach einem der vorangehenden Ansprüche, **dadurch gekennzeichnet, dass** das Ophthalmoskop (1) im ersten Zwischenbereich (19) zwischen dem ersten und dem zweiten Linsensystem (3, 13) keinen Spiegel, keinen Strahlteiler und/oder keine weitere Linse beinhaltet.

11. Ophthalmoskop (1) nach einem der vorangehenden Ansprüche, **dadurch gekennzeichnet, dass** das Ophthalmoskop (1) in einem Zwischenbereich (21) zwischen dem Auge (2) und dem ersten Linsensystem keinen Spiegel, keinen Strahlteiler und/oder keine weitere Linse umfasst.

12. Ophthalmoskop (1) nach einem der vorangehenden Ansprüche, **dadurch gekennzeichnet, dass** neben der Abbildung des Bereiches (4) im Auge (2) in der Zwischenbildebene (5) und in der Abbildungsebene (8) keine weiteren Abbildungen vorgesehen sind.

13. Verfahren zum Beobachten eines Auges (2), insbesondere eines Hintergrundes (15) des Auges, mit einem Ophthalmoskop (1), wobei ein im Auge (2) reflektierter Beobachtungsstrahl (17) durch ein erstes Linsensystem (3) des Ophthalmoskops (1) als Zwischenbild in einer Zwischenbildebene (5) abgebildet wird und das Zwischenbild (5) durch eine Abbildungsoptik (7) einer Beobachtungsvorrichtung (6) des Ophthalmoskops (1) in einer Abbildungsebene (8) abgebildet wird, wobei eine Apertur (9) der Abbildungsoptik (7) in einer Pupille (10) des Auges (2) abgebildet wird,
**dadurch gekennzeichnet,**
**dass** sich die Zwischenbildebene (5) innerhalb eines streuenden zweiten Linsensystems (13) in einem ersten Zwischenraum (19) zwischen dem ersten Linsensystem (3) und dem zweiten Linsensystem (13) in unmittelbarer Nähe zu einer dem ersten Linsensystem (3) zugewandten Oberfläche (13') des zweiten Linsensystems (13) oder in einem zweiten Zwischenraum (19') zwischen dem zweiten Linsensystem (13) und der Abbildungsoptik (7) in unmittelbarer Nähe zu einer der Abbildungsoptik (7) zugewandten Oberfläche (13'') des zweiten Linsensystems (13) befindet, wobei der Beobachtungsstrahl (17) in einem Strahlenverlauf zwischen dem ersten Linsensystem (3) und der Abbildungsoptik (7) durch das zweites Linsensystem (13) gestreut wird.

14. Verfahren aus Anspruch 13, **dadurch gekennzeichnet, dass** es mit einem Ophthalmoskop (1) nach einem der Ansprüche 1 bis 12 durchgeführt wird.

## Claims

1. An ophthalmoscope (1) for observing an eye (2), in particular a fundus (15) of the eye (2), the ophthalmoscope comprising a converging first lens system (3) for generating a real intermediate image of a region (4) in the eye (2) in an intermediate image plane (5) as well as an observation apparatus (6) having imaging optics (7) for imaging the real intermediate image in an imaging plane (8) in the observation apparatus (6) and configured for imaging an aperture (9) of the imaging optics (7) in a pupil (10) of the eye (2),
**characterized in that**
a second diverging lens system (13) is arranged between the first lens system (3) and the imaging optics (7) of the observation apparatus (6) in such a way that the intermediate image plane (5) is located within the second lens system (13) in a first interstice (19) between the first lens system (3) and the second lens system (13) in close proximity to a surface (13') of the second lens system (13) facing the first lens system (3) or in a second interstice (19') between the second lens system (13) and the imaging optics (7) in close proximity to a surface (13") of the second lens system (13) facing the imaging optics (7).

2. The ophthalmoscope (1) of claim 1, **characterized in that** the second lens system comprises at least two lenses, wherein the intermediate image plane is arranged in an interstice between the at least two lenses.

3. Ophthalmoscope (1) according to claim 1, **characterized in that** the second lens system (13) comprises exactly one diverging lens (13) and the intermediate image plane (5) is arranged within this lens (13).

4. Ophthalmoscope (1) according to claim 1, **characterized in that** the intermediate image plane (5) is arranged in the first interstice (19) at a distance of 1 mm to 20 mm to a surface (13') of the second lens system (13) facing the first lens system or that the intermediate image plane (5) is arranged in the second interstice (19') at a distance of 1 mm to 20 mm to a surface (13') of the second lens system facing the imaging optics (7) to reduce a radiation power within the second lens system (13) while simultaneously providing for a large working distance (A).

5. Ophthalmoscope (1) according to one of the preceding claims, **characterized in that** at least one lens (13) included in the second lens system (13) is tilted relative to the intermediate image plane (5) in order to suppress diffused light.

6. Ophthalmoscope (1) according to one of the preceding claims, **characterized in that** at least one lens included in the second lens system is tilted relative to the intermediate image plane (5) by an angle (β) in a range of between 0° and 45°.

7. Ophthalmoscope (1) according to one of the preceding claims, **characterized in that** the first lens system (3) comprises a lens (3) having a curved surface (3') that is aspherically shaped to improve imaging characteristics of the first lens system.

8. Ophthalmoscope (1) according to one of the preceding claims, **characterized in that** the second lens system (13) comprises a lens (13) having a curved surface (13') that is aspherically shaped to improve imaging characteristics of the second lens system.

9. Ophthalmoscope (1) according to one of the preceding claims, **characterized in that** the observation apparatus (6) comprises a light sensitive sensor (16) arranged in the imaging plane (8) for generating electric image signals.

10. Ophthalmoscope (1) according to one of the preceding claims, **characterized in that** the ophthalmoscope (1) includes no mirror, no beam splitter, and/or no further lens in the first intermediate space (19) between the first and the second lens system (3, 13).

11. Ophthalmoscope (1) according to one of the preceding claims, **characterized in that** the ophthalmoscope (1) does not include a mirror, a beam splitter, and/or further lens in a interstice (21) between the eye (2) and the first lens system.

12. Ophthalmoscope (1) according to one of the preceding claims, **characterized in that** no further images are provided in addition to the image of the region (4) in the eye (2) in the intermediate image plane (5) and in the imaging plane (8).

13. A method for observing an eye (2), in particular a fundus (15) of the eye, using an ophthalmoscope (1), wherein an observation beam (17) reflected in the eye (2) is imaged as an intermediate image in an intermediate image plane (5) by a first lens system (3) of the ophthalmoscope (1) and the intermediate image (5) is imaged an imaging plane (8) by imaging optics (7) of an observation apparatus (6) of the ophthalmoscope (1), wherein an aperture (9) of the imaging optics (7) is imaged in a pupil (10) of the eye (2),
**characterized in that** the intermediate image plane (5) is located within a second lens system (13) in a first interstice (19) between the first lens system (3) and the second lens system (13) in close proximity to a surface (13') of the second lens system (13) facing the first lens system (3) or in a second interstice (19') between the second lens system (13) and the imaging optics (7) in close proximity to a surface (13") of the second lens system (13) facing the imaging optics (7), wherein the observation beam (17) is diverged by the second lens system (13) in a beam path between the first lens system (3) and the imaging optics (7).

14. Method according to claim 13, **characterized in that** it is performed with an ophthalmoscope (1) according to one of the claims 1 to 12.

## Revendications

1. Ophtalmoscope (1) pour l'observation d'un œil (2), en particulier d'un fond (15) de l'œil (2), comprenant un premier système de lentilles collecteur (3) pour générer une image intermédiaire réelle d'une région (4) dans l' œil (2) dans un plan d'image intermédiaire (5) ainsi qu'un dispositif d'observation (6) avec un système optique d'imagerie (7) pour former l'image intermédiaire réelle dans un plan d'imagerie (8) dans le dispositif d'observation (6) et pour représenter une ouverture (9) du système optique d'imagerie (7) dans une pupille (10) de l' œil (2),
**caractérisé en ce qu'**un deuxième système dispersif de lentilles (13) est disposé entre le premier système de lentilles (3) et le système optique d'imagerie (7) du dispositif d'observation (6) de telle manière que le plan d'image intermédiaire (5) est situé à l'intérieur du deuxième système de lentilles (13) dans un premier espace intermédiaire (19) entre le premier système de lentilles (3) et le deuxième système de lentilles (13) à proximité immédiate d'une surface (13') du deuxième système de lentilles (13) disposée vis-à-vis du premier système de lentilles (3), ou dans un deuxième espace intermédiaire (19') entre le deuxième système de lentilles (13) et le système optique d'imagerie (7) à proximité immédiate d'une surface (13") du deuxième système de lentilles (13) disposée vis-à-vis du système optique d'imagerie (7).

2. Ophtalmoscope (1) selon la revendication 1, **caractérisé en ce que** le deuxième système de lentilles comprend au moins deux lentilles, le plan d'image intermédiaire étant situé dans un espace intermédiaire entre les au moins deux lentilles.

3. Ophtalmoscope (1) selon la revendication 1, **caractérisé en ce que** le deuxième système de lentilles (13) comprend exactement une lentille dispersive (13) et que le plan d'image intermédiaire (5) est disposé à l'intérieur de cette lentille (13).

4. Ophtalmoscope (1) selon la revendication 1, **caractérisé en ce que** le plan d'image intermédiaire (5) est disposé dans le premier espace intermédiaire (19) à une distance de 1 mm à 20 mm d'une surface (13') du deuxième système de lentilles (13) disposée vis-à-vis du premier système de lentilles, ou **en ce que** le plan d'image intermédiaire (5) est disposé dans le deuxième espace intermédiaire (19') à une distance de 1 mm à 20 mm d'une surface (13') du deuxième système de lentilles (13) disposée vis-à-vis du système optique d'imagerie (7) pour réduire une puissance rayonnée à l'intérieur du deuxième système de lentilles (13) avec en même temps une grande distance de travail (A).

5. Ophtalmoscope (1) selon l'une quelconque des revendications précédentes, **caractérisé en ce qu'**au moins une lentille (13) comprise dans le deuxième système de lentilles (13) est inclinée par rapport au plan d'image intermédiaire (5) pour supprimer la lumière diffusée.

6. Ophtalmoscope (1) selon l'une quelconque des revendications précédentes, **caractérisé en ce qu'**au moins une lentille comprise dans le deuxième système de lentilles est inclinée par rapport au plan d'image intermédiaire (5) d'un angle (β) compris entre 0° et 45°.

7. Ophtalmoscope (1) selon l'une quelconque des revendications précédentes, **caractérisé en ce que** le premier système de lentilles (3) comprend une lentille (3) ayant une surface courbe (3') de forme asphérique pour améliorer les caractéristiques de représentation du premier système de lentilles.

8. Ophtalmoscope (1) selon l'une quelconque des revendications précédentes, **caractérisé en ce que** le deuxième système de lentilles (13) comprend une lentille (13) ayant une surface courbe (13') de forme asphérique pour améliorer les caractéristiques de représentation du deuxième système de lentilles.

9. Ophtalmoscope (1) selon l'une quelconque des revendications précédentes, **caractérisé en ce que** le dispositif d'observation (6) comprend un capteur photosensible (16) disposé dans le plan d'imagerie (8) pour générer des signaux d'image électriques.

10. Ophtalmoscope (1) selon l'une quelconque des revendications précédentes, **caractérisé en ce que** l'ophtalmoscope (1) ne comprend aucun miroir, aucun séparateur de faisceau et/ou aucune autre lentille dans la première zone intermédiaire (19) entre le premier et le deuxième système de lentilles (3, 13).

11. Ophtalmoscope (1) selon l'une quelconque des revendications précédentes, **caractérisé en ce que** l'ophtalmoscope (1) ne comprend aucun miroir, aucun séparateur de faisceau et/ou aucune autre lentille dans une zone intermédiaire (21) entre l' œil (2) et le premier système de lentilles.

12. Ophtalmoscope (1) selon l'une quelconque des revendications précédentes, **caractérisé en ce qu'**aucune autre représentation n'est prévue dans le plan d'image intermédiaire (5) et dans le plan imagerie (8) outre la représentation de la zone (4) dans l'œil (2).

13. Méthode d'observation d'un œil (2), en particulier d'un fond (15) de l'œil, avec un ophtalmoscope (1), un faisceau d'observation (17) réfléchi dans l'œil (2) étant représenté par un premier système de lentilles (3) de l'ophtalmoscope (1) en tant qu'une image intermédiaire dans un plan d'image intermédiaire (5), et l'image intermédiaire (5) étant représentée par un système optique d'imagerie (7) d'un dispositif d'observation (6) de l'ophtalmoscope (1) dans un plan d'imagerie (8), une ouverture (9) du dispositif d'imagerie optique (7) étant représentée dans une pupille (10) de l'œil (2), **caractérisé en ce**
**que** le plan d'image intermédiaire (5) est situé à l'intérieur d'un deuxième système dispersif de lentilles (13) dans un premier espace intermédiaire (19) entre le premier système de lentilles (3) et le deuxième système de lentilles (13) à proximité immédiate d'une surface (13') du deuxième système de lentilles (13) disposée vis-à-vis du premier système de lentilles (3), ou dans un deuxième espace intermédiaire (19') entre le deuxième système de lentilles (13) et le système optique d'imagerie (7) à proximité immédiate d'une surface (13") du deuxième système de lentilles (13) disposée vis-à-vis du système optique d'imagerie (7), le faisceau d'observation (17) étant diffusé sur un tracé de faisceau entre le premier système de lentilles (3) et le système optique d'imagerie (7) par un deuxième système de lentilles (13).

14. Méthode selon la revendication 13, **caractérisée en ce qu'**elle est réalisée à l'aide d'un ophtalmoscope (1) selon l'une quelconque des revendications 1 à 12.
